# EUROPEAN PATENT APPLICATION

(11) **EP 4 574 015 A1**
(43) Date of publication of application: **25.06.2025**
(21) Application number: 24220971.6
(22) Date of filing: 18.12.2024
(51) Int. Cl.: A61B 3/00

(54) **FUNCTIONAL VISION HEAD IMPULSE TEST**

(30) Priority: 20.12.2023 US 202363612383 P
(71) Applicant: Interacoustics A/S, 5500 Middelfart (DK)
(72) Inventor: PETRAK, Michelle Renee, 5500 Middelfart (DK); KJÆRSGAARD, Lasse, 5500 Middelfart (DK); FUEMMELER, Elizabeth, 5500 Middelfart (DK)
(74) Representative: Demant

(57) **Abstract**

The present application relates to a system for evaluating the function of the vestibular system of a test subject. The system comprises an eye measurement device configured to record a pupil position, a velocity value, and/or an acceleration value of at least one of the eyes of the test subject, a motion sensing device configured to sense a movement and/or location of at least the head of the test subject and to provide at least one motion signal representing an acceleration value and/or a velocity value of a rotation of the head of the test subject in at least one plane, a display device configured to display at least one image, a user input device for receiving a user input, a processing device connected to the eye measurement device, the motion sensing device, and the display device, wherein the processing device is configured to execute at least one of a plurality of test protocols, where one test protocol comprises continuously analysing the at least one motion signal from said motion sensing device and determining a point in time with a maximum acceleration value, presenting said image, characterised by a property, for a pre-set time interval by said display device starting from the determined point in time with maximum acceleration value, receiving a user input comprising an estimate of the property of said image via the user input device, wherein the processing device is configured to determine a correspondence between said property and said estimate of said property.

## Description

### SUMMARY

The present application relates to a system for evaluating the function of the vestibular system of a test subject.

The present application further relates to a method of evaluating the function of the vestibular system of a test subject.

The present application further relates to a data processing system comprising a processing device and program code means for causing the processing device to perform at least some of the steps of the method.

The present application further relates to a computer program comprising instructions which, when the program is executed by a system, cause the system to carry out the steps of the method.

### A System

The Vestibular Ocular Reflex (VOR) is an eye movement that allows the gaze of the eyes of a test subject (i.e., of a patient) to stay focused on a target during head movements.

The VOR can be assessed with different techniques. The techniques include video goggles with camera(s) and accelerometer(s) attached, to measure both eye velocity and head velocity. From this a gain can be determined, which is eye velocity divided by head velocity. Other techniques use only a head mounted accelerometer to measure head velocity and rely on the test subject's responses to the presence of an Optotype on a screen in front of the test subject to measure visual acuity. Visual acuity is a functional measurement whereas gain is an objective mathematical measurement.

In other words, the purpose of VOR tests/techniques is to evaluate the function of the vestibular system (i.e., comprising vestibular semicircular canals and associated vestibular-ocular reflex (VOR)). Peripheral vestibular canal function may be tested with two types of measurements (1) "conventional" video head impulse tests (vHIT) measure angular VOR (aVOR) gain - estimates reduced gain and corrective eye movement, and (2) more recently functional gain is assessed using visual acuity as a measure of how well the aVOR performs in stabilizing gaze during head movements. vHIT is performed passively, as the clinician (i.e., the user operating the system) moves the head. Functional tests have traditionally been passive also where the clinician moves the test subject's head. However, for example, active tests where the test subjects can move their own head to the sound of a metronome (or in response to a clinician direction) to generate VOR gains are known. This may be a better measurement of the function of the vestibular system since everyday living situations involve the test subjects moving their own head actively. However, none of these tests provide a determination of the VOR function at maximum head acceleration as existing systems are not able to display the optotype, only briefly, at maximum head accelerations.

Accordingly, there is a need for providing a system suitable for determining the VOR function at maximum acceleration.

In an aspect of the present application, a system for evaluating the function of the vestibular system of a test subject is provided.

An apparatus or instrument comprising all of the elements of the system is furthermore provided.

The system may comprise an eye measurement device.

The eye measurement device may be configured to record (such as measure and store) a pupil position of at least one of the eyes of the test subject.

The eye measurement device may be configured to generate a pupil position signal. The pupil position signal may comprise information regarding a velocity value and/or an acceleration value of at least one of the eyes of the test subject.

The eye measurement device may be configured to record the velocity value of at least one of the eyes of the test subject.

The eye measurement device may be configured to record the acceleration value of at least one of the eyes of the test subject.

Pupil position may refer to the location or orientation of the eye(s) of the test subject.

At least one eye may refer to the one or both eyes of the test subject.

For example, from the pupil position of at least one of the eyes of the test subject, the system (or eye measurement device) may be configured to determine the direction of eyesight (or the focus point of the eye(s)) relative to a display.

For example, the eye measurement device may be a binocular camera system.

For example, the eye measurement device may be (video) goggles comprising a recording device, such as a camera.

The system may comprise a motion sensing device.

The motion sensing device may be configured to sense a movement of at least the head of the test subject.

The motion sensing device may be configured to sense a location of at least the head of the test subject.

The motion sensing device may be configured to provide at least one motion signal representing an acceleration value of a rotation of the head of the test subject in at least one plane.

Additionally, or alternatively, the at least one motion signal may represent a velocity value of a rotation of the head of the test subject in at least one plane.

Acceleration value may refer to a size of the acceleration represented in degrees per second² (°/s²) and velocity value may refer to a size of the velocity represented in degrees per second (°/s).

For example, the motion sensing device may be a 9 degrees of freedom (DOF) inertial measurement unit (IMU) sensor.

The system may comprise a display device.

The display device may be configured to display at least one image.

For example, the display device may be a PC monitor or screen.

The system may comprise a user input device.

The user input device may be suitable for (configured to) receiving a user input.

For example, the user input may be provided by a user of the system (e.g., an examiner and/or a clinician) or may be provided by the test subject. For example, the user input may be the user or test subject pressing a key or switch on the user input device.

The system may comprise a processing device.

The processing device may be connected to the eye measurement device, the motion sensing device, and the display device. The processing device may further be connected to the user input device.

The term 'connected to' may refer to that the processing device is operationally "connected" or "coupled" to the other elements. In other words, the processing device can be directly connected or coupled to the other elements, but an intervening additional element (such as a transmitting/receiving unit) may also be present, unless expressly stated otherwise. Furthermore, "connected" or "coupled" as used in the present application may include wirelessly connected or coupled. For example, the processing device may be configured to send and/or receive control signals/information/data via the connection.

The processing device may be configured to execute at least one of a plurality of test protocols. The processing device may be configured to execute and/or initiate at least one of a plurality of test protocols in response to receiving an activation/initiation signal (e.g., a user input via the user input device).

One test protocol (of said plurality of test protocols, e.g., comprising a Video Head Impulse Test (vHIT) test protocol, a Functional Vision Head Impulse Test (fvHIT) test protocol, an Active Head Rotation (AHR) test protocol, a Dynamic Visual Acuity (DVA) test protocol, a Gaze Stabilization Test (GST) test protocol, and/or a Visual Process Time (VPT) test protocol) may comprise a step of continuously analysing the at least one motion signal from said motion sensing device and determining a point in time with a maximum acceleration value.

The one test protocol may further comprise presenting said image, characterised by a property, for a pre-set time interval by said display device starting from the determined point in time with maximum acceleration value.

For example, the pre-set time interval may be at least 30 ms.

For example, the pre-set time interval may be less than 100 ms.

For example, the pre-set time interval (or the minimum time needed to present the image) may be determined by the VPT test prior to carrying out the test protocol.

The one test protocol may further comprise receiving a user input comprising an estimate of the property of said image via the user input device.

Accordingly, the plurality of test protocols may comprise an fvHIT protocol comprising at least:
- continuously analysing the at least one motion signal from said motion sensing device and determining a point in time with a maximum acceleration value,
- presenting said image, characterised by a property, for a pre-set time interval by said display device starting from the determined point in time with maximum acceleration value,
- receiving a user input comprising an estimate of the property of said image via the user input device.

The processing device may be configured to determine a correspondence between said property and said estimate of said property.

Thereby, a system suitable for determining the VOR function at maximum acceleration is provided.

The display device may be configured to display the at least one image.

Thereby, the image may be visible to the test subject.

The at least one image may comprise an optotype.

The at least one image may comprise one of a plurality of optotypes.

The display device may be configured to display one of said plurality of optotypes in response to receiving a user input via the user input device.

Thereby, the user may choose the image (e.g., the optotype) most suitable for the test protocol to be carried out.

The property of said at least one image may relate to an orientation of said image.

For example, the image may be a Landolt C optotype.

For example, the image may be a Tumbling E optotype.

For example, the image may be a VisualEyes C optotype.

The optotype may then be oriented towards the left, right, top, or bottom part of the display device.

The property of said at least one image may relate to a size of said image.

The property of said at least one image may relate to a location of said image.

For example, the image may be displayed at different locations on the display device, such as in the centre or at one of the edges of the display device.

The property of said at least one image may relate to a contrast level of said image.

The step of continuously analysing the at least one motion signal from said motion sensing device and determining a point in time with the maximum acceleration value, may comprise that the processing unit:
- compares the acceleration value of each of the at least one motion signal with a pre-determined acceleration threshold.

For example, the pre-determined acceleration threshold may be at least 750°/s². Thereby, the pre-determined acceleration threshold may e.g., be 750°/s², 800°/s², 850°/s², 900°/s², etc., or any value in between.

The step of continuously analysing the at least one motion signal from said motion sensing device and determining a point in time with the maximum acceleration value, may comprise that the processing unit:
- compares the velocity value of each of the at least one motion signal with a pre-determined velocity threshold.

For example, the pre-determined velocity threshold may be at least 40°/s. Thereby, the pre-determined velocity threshold may e.g., be 40°/s, 45°/s, 50°/s, 55°/s, etc., or any value in between.

The step of continuously analysing the at least one motion signal from said motion sensing device and determining a point in time with the maximum acceleration value, may comprise that the processing unit:
- presents a start signal, in response to both the acceleration and velocity values exceed their respective thresholds.

The start (initiation) signal may be presented at an earlier point in time than at the point in time with maximum acceleration value. In other words, a time delay may exist between said earlier point in time and said point in time with maximum acceleration value. The time delay may e.g., be at least 20 ms, such as 30 ms.

For example, the start signal may represent an estimated point in time before the acceleration value reaches its actual maximum. In other words, the start signal may be presented with a time delay before the (estimated time of) maximum acceleration value.

The time delay may represent the latency of the system, e.g., of the graphics part of the system, such as in the software handling the display of the image and in the display device.

The step of presenting said image, characterised by a property, for a pre-set time interval by said display device starting from the determined point in time with maximum acceleration value, may comprise:
- presenting said image by the display device, in response to said start signal being presented by the processing unit.

The property of said image may be determined by the executed test protocol.

The step of receiving a user input comprising an estimate of the property of said image via the user input device may comprise:
- receiving a response from the test subject or from the user of the system.

In other words, when the test subject's head is rotated at maximum acceleration, an image (e.g., an optotype) may be displayed on the display unit for the test subject to see. Then, either the test subject or the user of the system may provide a user input to the system (via the user input device) comprising an estimate of the property of the image, which could for example comprise an indication of the orientation of the image.

The processing device may be configured to score the percentage of correct estimates (i.e., from the test subject) of said property at different accelerations of rotation of the head of the test subject in each respective plane.

The processing device may be configured to re-execute a specific test protocol in response to receiving an instruction in form of a user input via said user input device.

For example, when a specific test protocol has been carried out, e.g., in which the test subjects' head has been rotated and optotypes displayed at maximum head acceleration, the clinician may want to repeat the test protocol. This may be initiated in response to the clinician providing a user input, e.g., instructions/control signal to repeat the test protocol.

The processing device may be configured to execute one of the plurality of test protocols in response to receiving an instruction to execute a specific one of the plurality of test protocols via the user input device.

Thereby, the user may design which test protocol to be executed when, and if one or more test protocols should be repeated.

The at least one plane may comprise a lateral plane.

The at least one plane may comprise a Right Anterior, Left Posterior (RALP) plane.

The at least one plane may comprise a Left Anterior, Right Posterior (LARP) plane.

The at least one plane may comprise a vertical plane.

The lateral plane may refer to a head rotation in a horizontal plane. For example, the test subject's head may be bowed forward by approximately 30°. Thereby, the horizontal canals of each ear (which are a co-planar pair) are stimulated the best.

The RALP plane may refer to a head rotation in an orientation which best stimulates the Right Anterior, Left Posterior co-planar pair.

The LARP plane may refer to a head rotation in an orientation which best stimulates the Left Anterior, Right Posterior co-planar pair.

The motion sensing device may be configured to be attached to the eye measurement device.

The motion sensing device may be releasably attached to the eye measurement device.

Thereby, the motion sensing device may be attached before execution of a specific test protocol, and be detached again afterwards if not relevant for the following test protocol.

Alternatively, the motion sensing device may be arranged at the head of the test subject by use of a headband, and thereby not being directly connected to the eye measurement device.

For example, the motion sensing device may comprise a single motion sensor, such as an accelerometer, which may be located at a central position of the eye measurement device, such as at the forehead of test subject. Alternatively, the motion sensing device may comprise two or more motion sensors, such as two accelerometers, which may be located e.g., near each of the eyes of the test subject.

The display device may comprise a refresh rate of at least 240 Hz.

Refresh rate may refer to a scan rate of the display device, such as a scan rate of a monitor.

For example, the refresh rate may be even higher, as this will only increase the reliability of displaying of the image at the correct moment.

The user input device may comprise a remote control.

A remote control may make it easy for the test subject to provide his/her estimate of the property of the image.

The user input device may comprise a keyboard.

The user input device may comprise a touch screen.

The processing device may be configured to execute at least one of a plurality of test protocols.

The plurality of test protocols may comprise the AHR test protocol.

For example, the system may comprise the eye measurement device, e.g., a goggle with a camera configured to record at least one of the eyes of the test subject. The system may further comprise a motion sensing device, e.g., an accelerometer worn on the head of the test subject. The motion sensing device may be attached to the eye measurement device. The system may further comprise a processing device configured to execute the AHR test protocol.

The AHR test protocol may comprise (at a plurality of head rotation velocity values):
- presenting said image, characterised by a property, by said display device,
- continuously analysing the at least one motion signal from said motion sensing device and determining a velocity value of a rotation of the head of the test subject,
- continuously determining a velocity value of at least one of the eyes of the test subject, by the eye measurement device,
- determining a gain value by dividing eye velocity with head velocity.

For example, the head may be rotated at multiple different frequencies. The frequencies may e.g., be 1-3 Hz or higher. In other words, the AHR test protocol may comprise that the user/clinician moves the test subject's head left or right with a controlled sinusoidal movement timed with a metronome. At the same time, a fixed image may be displayed by the display device. The processing device may measure gain which is eye velocity divided by head velocity (and also phase and asymmetry) at different head rotation frequencies.

The plurality of test protocols may comprise the vHIT test protocol.

For example, the system may comprise the eye measurement device, e.g., a goggle with a camera recording at least one of the eyes of the test subject. The system may further comprise a motion sensing device, e.g., an accelerometer worn at the head of the test subject. The motion sensing device may be attached to the eye measurement device. The system may further comprise a processing device configured to execute the vHIT test protocol.

The vHIT test protocol may comprise:
- presenting said image, characterised by a property, by said display device,
- continuously analysing the at least one motion signal from said motion sensing device and determining a velocity value of a movement of the head of the test subject,
- continuously determining the velocity value of at least one of the eyes of the test subject, by the eye measurement device,
- determining a gain value by dividing eye velocity value with head velocity value.

For example, the head may be moved in a fast impulse movement. For example, the fast impulse movement may be +/- 15 degrees relative to when the head is at a resting position.

In other words, the vHIT test protocol may comprise that the user/clinician quickly moves the test subject's head left or right in the lateral plane with a high-speed impulse movement, or in the RALP plane or LARP plane. At the same time, a fixed image may be displayed by the display device. The processing device may measure gain which is eye velocity value divided with head velocity value.

The plurality of test protocols may comprise the DVA (or DVAT) test protocol.

For example, the system may comprise a motion sensing device, e.g., an accelerometer worn at the head of the test subject. The system may further comprise a processing device configured to execute the DVA test protocol.

The DVA test protocol may comprise:
- continuously analysing the at least one motion signal from said motion sensing device and determining a velocity value of a rotation of the head of the test subject,
- in response to the velocity value reaching and/or exceeding a threshold velocity value, presenting said image, characterised by a property, by said display device,
- receiving a user input comprising an estimate of the property of said image via the user input device,
- determining a correspondence between said property and said estimate of said property.

The DVA test protocol may further comprise:
- in response to said correspondence between said property and said estimate of said property exceeds a correspondence threshold, comparing the size of said image to the size of said image determined at a velocity value of 0°/s (i.e., as determined by a static DVA test protocol).

For example, the threshold velocity value may be at least 50°/s and/or less than 300°/s. For example, the threshold velocity value may be 100°/s.

In other words, the DVA test protocol may comprise that the user/clinician moves the test subject's head left and right (in the horizontal plane) or up and down (in the vertical plane) with a controlled sinusoidal movement timed with a metronome. The test subject may view an image, e.g., an optotype, that appears on the display device when a certain head velocity is reached, and the test subject may in response tell the direction the optotype is pointing. The processing device may in response determine (e.g., keep score of) the change in optotype size that is needed for the test subject to clearly see the optotype while the head is at maximum velocity and compare this determined optotype size to the optotype size needed to see it clearly when the head is not moving. The needed optotype size, when the head is not moved, may be determined by reducing the optotype in size until a threshold is found.

The plurality of test protocols may comprise the GST test protocol.

For example, the system may comprise a motion sensing device, e.g., an accelerometer worn at the head of the test subject, and a display device configured to display at least one image. The system may further comprise a processing device configured to execute the GST test protocol.

The GST test protocol may comprise:
- continuously analysing the at least one motion signal from said motion sensing device and determining a velocity value of a rotation of the head of the test subject,
- in response to the velocity value reaching and/or exceeding a threshold velocity value, presenting said image, characterised by a property, by said display device,
- receiving a user input comprising an estimate of the property of said image via the user input device,
- determining a correspondence between said property and said estimate of said property.

The GST test protocol may further comprise:
- keeping score of the velocity values, and
- determining the highest velocity value at which there is correspondence between said property and said estimate of said property.

For example, the property of said at least one image may relate to an orientation of said image. For example, the velocity value may be at least 50°/s, and/or less than 300°/s.

In other words, the GST test protocol may comprise that the user/clinician/examiner moves the test subject's head left and right (in the horizontal plane) or up and down (in the vertical plane) with a controlled sinusoidal movement timed with a metronome at 50-300°/s. The test subject may view an image, e.g., an optotype, that appears on the display device when a certain velocity value (head speed) is reached, and the test subject may in response tell the direction the image is pointing. The processing device (e.g., the software) may in response determine (keeps score) of the velocity values and determine the highest velocity value at which the image is still clear to test subject. For example, the size of the image may be the same, but the velocity value may change.

The plurality of test protocols may comprise a VPT test protocol.

The VPT test protocol may comprise.
- presenting an image, characterised by a property, on the display device for a pre-set time interval,
- receiving a user input comprising an estimate of the property of said image via the user input device,
- determining a correspondence between said property and said estimate of said property,
- in response to correspondence between said property and said estimate, lowering the time interval of presenting said image, and
- determining the minimum time interval at which there is correspondence between said property and said estimate.

In other words, the VPT test protocol may comprise displaying an optotype on a screen at different time intervals (between 30 ms and 100 ms). The test (the pre-set time interval) starts around 70-80 ms and if the test subject correctly identifies the optotype, a thresholding process begins to find the fastest optotype the test subject can correctly identify.

The processing device may be configured to determine a visual focus of the test subject on said display device.

The visual focus may be determined based on the recorded orientation of at least one of the eyes of the test subject.

Recorded orientation may refer to the orientation of the eyes of the test subject relative to the display device.

The processing device may be configured to determine an average response rate calculation.

For example, the average response rate calculation may comprise the processing device being configured to calculate the correct response rate per acceleration bin, i.e., the ratio of correct responses per acceleration bin. The acceleration bins may be within 2000-7000 °/s, e.g., the bins may be divided into 1000-2000 °/s, 2000-3000 °/s, 3000-4000 °/s, 4000-5000 °/s, 5000-6000 °/s, and 6000-7000 °/s. The correct response rate per acceleration bin may be calculated for one or more planes of rotation/movement of the head of the test subject.

For example, the processing device may be configured to provide an average response rate across all acceleration bins (per plane of rotation/movement).

The system may further comprise a timer.

The system may be configured to time the duration from presenting an image until receiving a user input.

The system may be configured to implement a (predetermined) time interval from presenting said image until the processing device presents a predetermined response. For example, the predetermined response may indicate that no response will be provided, such as "I don't know", so that the user does not have to select an answer on the computer or through the remote. In other words, the system may be configured to record a "no response" user input, in case the user does not provide a user input within the (predetermined) time interval.

For example, the (predetermined) time interval may be 5 seconds, or more.

The system may be configured to provide (real time) feedback to the user during execution of at least one of said plurality of test protocols.

For example, said feedback may be provided on a test screen, e.g., which may be visible to the user/operator of the system (e.g., an examiner and/or a clinician).

For example, the feedback may be represented by the acceleration bins at the top of the test screen (e.g., an additional display device).

The system may comprise a room recording camera (e.g., an external camera attached to a computer (or built into the computer) of the system).

The system may be configured to record the patient during the test protocol, via said room recording camera. This may be beneficial to see head movements through video recording and match with test findings/evaluation.

The system may be configured to record audio during the test protocol, via said room recording camera. Thereby, audio for patient reactions/symptoms noted during the test may be recorded.

The eye measurement device may comprise a camera for recording said at least one eye of the test subject.

In a further aspect, the system further comprising an auxiliary device is moreover provided.

The system may be adapted to establish a communication link between the elements of the system (e.g., the eye measurement device, the motion sensing device, the display device, the user input device, the processing device, the auxiliary device, etc.) to provide that information (e.g., control and/or status signals, motion signals, start signals, etc.) can be exchanged or forwarded from one to the other.

The auxiliary device may comprise a remote control, a mobile device, a smartphone, or other portable electronic device, such as a tablet or the like, or may comprise a server device, etc.

The communication link may be a wired or wireless link, e.g., based on Bluetooth or some other standardized scheme.

### Use

In an aspect, use of a system as described above, and in the claims, is moreover provided.

### A method

In an aspect, a method of evaluating the function of the vestibular system of a test subject is furthermore provided by the present application.

The method may comprise executing at least one of a plurality of test protocols by a processing device.

One test protocol (of the plurality of test protocols) may comprise:
- continuously analysing at least one motion signal from a motion sensing device representing an acceleration of a rotation of the head of the test subject in at least one plane.

The one test protocol may comprise:
- determining a point in time with a maximum acceleration value.

The one test protocol may comprise presenting an image, characterised by a property, for a pre-set time interval by a display device starting from the determined point in time with maximum acceleration value.

The one test protocol may comprise:
- receiving a user input comprising an estimate of the property of said image via a user input device.

The method may further comprise determining a correspondence between said property and said estimate of said property.

The method of evaluating the function of the vestibular system of the test subject may comprise executing at least one of a plurality of test protocols by the processing device, where one test protocol comprises the AHR test protocol (at a plurality of head rotation velocity values):
- presenting said image, characterised by a property, by said display device,
- continuously analysing the at least one motion signal from said motion sensing device and determining a velocity value of a rotation of the head of the test subject,
- continuously determining a velocity value of at least one of the eyes of the test subject, by the eye measurement device,
- determining a gain value by dividing eye velocity with head velocity.

The method may further comprise:
- rotating the head of the test subject in at least one plane in a sinusoidal movement at a constant velocity value of the head.

For example, the head may be rotated at multiple different frequencies. The frequencies may e.g., be 1-3 Hz or higher.

In other words, the AHR test protocol may comprise that the user/clinician moves the test subject's head left or right with a controlled sinusoidal movement timed with a metronome. At the same time, a fixed image may be displayed by the display device. The processing device may measure gain which is eye velocity divided by head velocity (and also phase and asymmetry) at different head rotation frequencies.

The method of evaluating the function of the vestibular system of the test subject may comprise executing at least one of a plurality of test protocols by the processing device, where one test protocol comprises the vHIT test protocol:
- presenting said image, characterised by a property, by said display device,
- continuously analysing the at least one motion signal from said motion sensing device and determining a velocity value of a movement of the head of the test subject,
- continuously determining the velocity value of at least one of the eyes of the test subject, by the eye measurement device,
- determining a gain value by dividing eye velocity value and head velocity value.

The method may further be characterised by head movements of high velocity, low amplitude and being unexpected.

For example, the head may be moved in a fast impulse movement. For example, the fast impulse movement may be +/- 15 degrees relative to when the head is at a resting position.

In other words, the vHIT test protocol may comprise that the user/clinician quickly moves the test subject's head left or right in the lateral plane with a high-speed impulse movement, or in the RALP plane or LARP plane. At the same time, a fixed image may be displayed by the display device. The processing device may measure gain which is eye velocity value divided with head velocity value.

The method of evaluating the function of the vestibular system of the test subject may comprise executing at least one of a plurality of test protocols by the processing device, where one test protocol comprises the DVA test protocol:
- continuously analysing the at least one motion signal from said motion sensing device and determining a velocity value of a rotation of the head of the test subject,
- in response to the velocity value reaching and/or exceeding a threshold velocity value, presenting said image, characterised by a property, by said display device,
- receiving a user input comprising an estimate of the property of said image via the user input device,
- determining a correspondence between said property and said estimate of said property.

The DVA test protocol may further comprise:
- in response to said correspondence between said property and said estimate of said property exceeds a correspondence threshold, comparing the size of said image to the size of said image at a velocity value of 0°/s.

The method may further comprise:
- rotating of the head of the test subject in at least one plane in a sinusoidal movement at a constant (head) velocity value.

For example, the threshold velocity value may be at least 50°/s and/or less than 300°/s. For example, the threshold velocity value may be 100°/s.

In other words, the DVA test protocol may comprise that the user/clinician moves the test subject's head left and right (in the horizontal plane) or up and down (in the vertical plane) with a controlled sinusoidal movement timed with a metronome. The test subject may view an image, e.g., an optotype, that appears on the display device when a certain head velocity is reached, and the test subject may in response tell the direction the optotype is pointing. The processing device may in response determine (e.g., keep score of) the change in optotype size that is needed for the test subject to clearly see the optotype while the head is at maximum velocity and compare this determined optotype size to the optotype size needed to see it clearly when the head is not moving. The needed optotype size, when the head is not moving, may be determined by reducing the optotype in size until a threshold is found.

The method of evaluating the function of the vestibular system of the test subject may comprise executing at least one of a plurality of test protocols by the processing device, where one test protocol comprises the GST test protocol:
- continuously analysing the at least one motion signal from said motion sensing device and determining a velocity value of a rotation of the head of the test subject,
- in response to the velocity value reaching and/or exceeding a threshold velocity value, presenting said image, characterised by a property, by said display device,
- receiving a user input comprising an estimate of the property of said image via the user input device,
- determining a correspondence between said property and said estimate of said property.

The GST test protocol may further comprise:
- keeping score of the velocity values, and
- determining the highest velocity value at which there is correspondence between said property and said estimate of said property.

For example, the property of said at least one image may relate to an orientation of said image. The method may further comprise:
- rotating of the head of the test subject in at least one plane in a sinusoidal movement at a velocity value.

For example, the velocity value may be at least 50°/s, and/or less than 300°/s.

In other words, the GST test protocol may comprise that the user/clinician/examiner moves the test subject's head left and right (in the horizontal plane) or up and down (in the vertical plane) with a controlled sinusoidal movement timed with a metronome at 50-300°/s. The test subject may view an image, e.g., an optotype, that appears on the display device when a certain velocity value (head speed) is reached, and the test subject may in response say the direction he/she believes the image is pointing. The processing device (e.g., the software) may in response determine (keeps score) of the velocity values and determine the highest velocity value at which the image is still clear to test subject. For example, the size of the image may be the same, but the velocity value may change.

It is intended that some or all of the structural features of the system described above, or in the claims can be combined with embodiments of the method, when appropriately substituted by a corresponding process and vice versa. Embodiments of the method have the same advantages as the corresponding system.

### A computer readable medium or data carrier

In an aspect, a tangible computer-readable medium (a data carrier) storing a computer program comprising program code means (instructions) for causing a data processing system (a computer) to perform (carry out) at least some (such as a majority or all) of the (steps of the) method described above, and in the claims, when said computer program is executed on the data processing system is furthermore provided by the present application.

### A computer program

A computer program (product) comprising instructions which, when the program is executed by a device (such as a system or an element of the system), cause the device to carry out (steps of) the method described above, and in the claims is furthermore provided by the present application.

### A data processing system

In an aspect, a data processing system comprising a processing device and program code means for causing the processing device to perform at least some (such as a majority or all) of the steps of the method described above, and in the claims is furthermore provided by the present application.

### An APP

In a further aspect, a non-transitory application, termed an APP, is furthermore provided by the present disclosure. The APP may comprise executable instructions configured to be executed, e.g., by the system, or on the elements of the system, or on the auxiliary device, such as on a display device or on a user input device, e.g., to implement a control interface (user interface).

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
FIG. 1 shows an example of a system of the present application.
FIG. 2 shows an example of the lateral plane, the RALP plane, the LARP plane, and the vertical plane of the present application.
FIG. 3 shows an example of determination of a point in time with a maximum acceleration value.
FIG. 4A shows an example of a flow diagram of a method of evaluating the function of the vestibular system of a test subject.
FIG. 4B shows an example of an additional flow diagram of a method of evaluating the function of the vestibular system of a test subject.
FIG. 5 shows an example of a flow diagram of a method of evaluating the function of the vestibular system of a test subject comprising a combination of test protocols.
FIG. 6 shows an example of an average response rate calculation.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

### DETAILED DESCRIPTION OF EMBODIMENTS

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the system and method are described by various blocks, functional units, modules, components, steps, processes, algorithms, etc. (collectively referred to as "elements"). Depending upon the particular application, design constraints or other reasons, these elements may be implemented using electronic hardware, computer program, or any combination thereof.

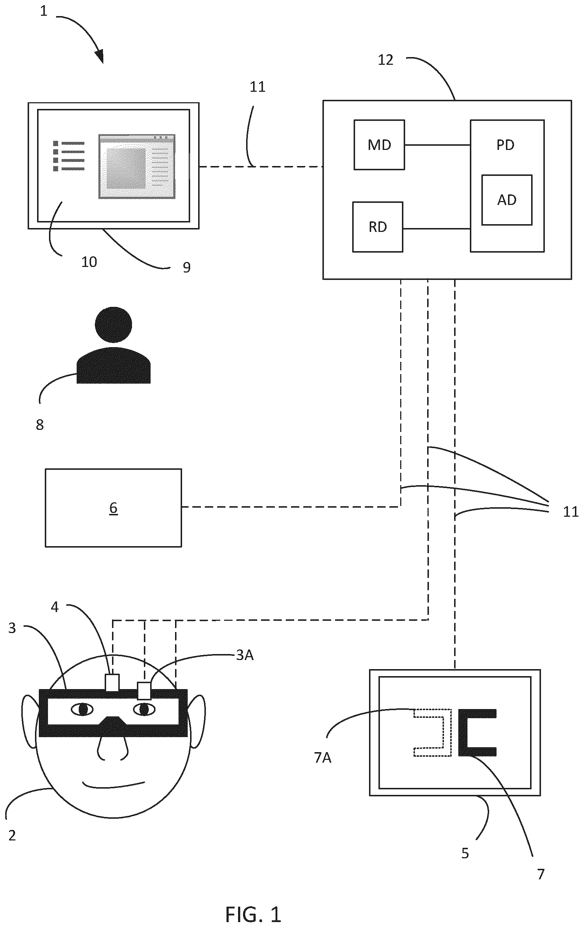

FIG. 1 shows an example of a system of the present application. The system 1 may be suitable for evaluating the function of the vestibular system of a test subject 2.

In FIG. 1, it is shown that the system 1 may comprise an eye measurement device 3, a motion sensing device 4, a display device 5, a user input device 6, and a processing device PD.

The eye measurement device 3 may be configured to record a pupil position, a velocity value, and/or an acceleration value of at least one of the eyes of the test subject 2. For example, the eye measurement device 3 may comprise a recording device 3A, e.g., a camera, for recording said at least one of the eyes of the test subject 2. In FIG. 1, the recording device 3A is shown to be located at only one of the eyes of the test subject 2, but it is foreseen that it may be located at any other location as long as it is configured to record the pupil position, velocity value, and/or acceleration value of one or both of the eyes of the test subject 2. As shown in FIG. 1, the eye measurement device 3 may be (video) goggles comprising the recording device 3A.

The motion sensing device 4 may be configured to sense a movement and/or location of at least the head of the test subject 2 and to provide at least one motion signal representing an acceleration value and/or a velocity value of a rotation of the head of the test subject 2 in at least one plane. The motion sensing device 4 is illustrated to be located at a centre/middle part of the eye measurement device 3, but may be located at any position of the eye measurement device 3, e.g., in a releasable manner. Alternatively, the motion sensing device 4 may be located at the head of the test subject 2 by use of an attachment means, e.g., by use of a head band, so that it is not dependent on the eye measurement device 3 for being fixed to the head of the test subject 2. For example, the motion sensing device 4 may be an accelerometer. The motion sensing device 4 may comprise two or more accelerometers for sensing the movement and/or location of the head of the test subject 2.

The display device 5 may be configured to display at least one image 7, which in FIG. 1 is exemplified as an optotype in form of the Landolt C optotype. The image 7 may be characterized by a property, which may refer to one or more of an orientation, a size, a location, or a contrast level of said image 7. In other words, the image 7 may be oriented towards the left, right, bottom, or top of the display device 5, or be displayed with varying sizes, be located either at the middle or along one of the edges of the display device 5, or be showed with varying contrast levels. The display device 5 may for example be a screen/monitor.

The user input device 6 may be configured to receive a user input. The user input may be provided by either the test subject 2 or by the user 8 of the system 1, which may be an examiner/clinician. For example, the user input may relate to the test subject's 2 estimate of the property of the image 7 presented on the display device 5. Alternatively, or additionally, the user input may relate to controlling the processing device PD, e.g., by sending instructions on which test protocol to execute, and/or to activate or deactivate the processing device PD.

As shown in FIG. 1, the system may additionally comprise an additional display device 9, which may be configured to present a user interface 10, e.g., for the user 8 to follow and control the test protocol.

The processing device PD is shown to be connected to the eye measurement device 3 (and/or may be connected directly to the recording device 3A), the motion sensing device 4, the display device 5, and the user input device 6. Further, the processing device PD may be connected to the additional display device 9 (e.g., a test screen). As indicated by the dashed lines, the connections 11 may be via a wired or wireless connection 11. The connection 11 may run via a receiving device RD, which may lead control signals/instructions to and from the processing device PD.

The processing device PD may be configured to execute at least one of a plurality of test protocols. For example, the test protocols may be started by the user 8 entering instructions (user input) via the user input device 6. Each of the test protocols may be stored in a memory device MD of the system 1. The user 8 may follow and control the execution of the test protocol on the user interface 10.

One of the plurality of test protocol, in particular the fvHIT test protocol, may comprise continuously analysing the motion signals transmitted via the connection 11 from the motion sensing device 4 to an analysis device AD of the processing device PD. Thereby, a point in time with a maximum acceleration value may be determined. In response to determination of said point in time, the display device 5 may present an image 7, e.g., in form of the Landolt C optotype, for a pre-set time interval from the determined point in time. The property of the image 7 may be set/determined by the processing device PD based on the test protocol. In response to viewing the presented image 7, the test subject 2 (or alternatively the user 8) may provide an input to the user input device 6 relating to the test subject's 2 estimate of the property of said image 7. For example, the property may relate to the orientation of the image 7, in other words, which way the image 7 is orientated. As an example, it is shown that the image 7 may alternatively be mirrored 7A and thereby be oriented in the opposite direction. Potentially, already received user input may be taken into account, when setting/determining the property of the image 7.

The processing device PD (or the analysis device AD) may be configured to determine a correspondence between the property of the image 7 and the test subject's 2 estimate of the property of the image 7. Correspondence may refer to whether or not the actual property and the estimated property are similar. The correspondence may be determined and stored at a number of maximum acceleration values, and be taken into account when evaluating the function of the vestibular system of the test subject 2. The determined correspondence and maximum acceleration values may be presented to and be evaluated by the user 8 on the user interface 10.

As shown in FIG. 1, the processing device PD, analysis device AD, memory device MD, and receiving device RD may be assembled in a housing 12.

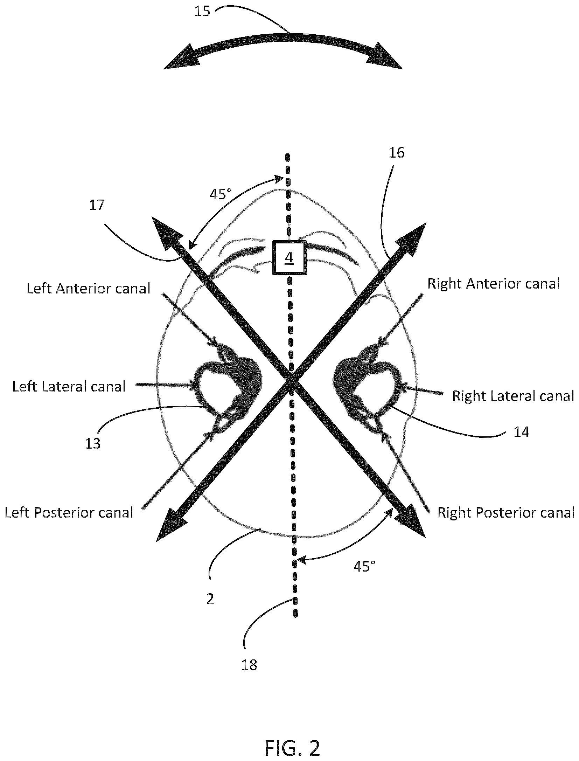

FIG. 2 shows an example of the lateral plane, the vertical plane, the RALP plane, and the LARP plane of the present application.

FIG. 2 shows the head of the test subject 2 seen from above. The vestibular system 13 at the left ear and the vestibular system 14 at the right ear are illustrated. The vestibular system 13 at the left ear comprises a left anterior canal, a left lateral canal, and a left posterior canal. The vestibular system 14 at the right ear comprises a right anterior canal, a right lateral canal, and a right posterior canal.

The motion sensing device 4 may be arranged at the forehead of the test subject 2, e.g., intersecting a vertical plane 18 dividing the head into a left and a right part. The motion sensing device 4 may be configured to sense a movement and/or location of the head of the test subject 2 and provide motion signals representing an acceleration value and/or a velocity value of a rotation of the head in at least one of the planes mentioned above. In other words, the motion sensing device 4 may sense in which plane and/or at which velocity/acceleration the user (not shown) moves/rotates the head of the test subject 2. The planes may be a lateral plane, a vertical plane, a RALP plane, and/or a LARP plane.

The lateral plane 15 may refer to a head rotation in a horizontal plane (perpendicular to the vertical plane 18). Usually, the head may be bowed forward by approximately 30°, as this best stimulates the horizontal canals of each ear (which are a co-planar canal pair), i.e., the left and right lateral canals.

The RALP plane 16 may refer to a head rotation in an orientation which best stimulates the Right Anterior, Left Posterior co-planar canal pair. The RALP plane 16 may be located at a 45° angle relative to the vertical plane 18.

The LARP plane 17 may refer to a head rotation in an orientation which best stimulates the Left Anterior, Right Posterior co-planar canal pair. The LARP plane 17 may be located at a 45° angle relative to the vertical plane 18.

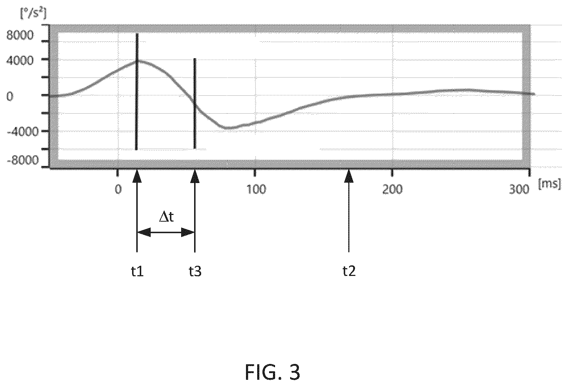

FIG. 3 shows an example of a determination of a point in time with a maximum acceleration value.

FIG. 3 depicts a graph with an example of the rotation of the head of the test subject in the lateral plane as measured by the motion sensing device and provided as motion signals. In the graph, the acceleration value (°/s²) of the rotation is shown as a function of time (ms) from a starting point to an end point of the rotation. As seen, the acceleration value initially increases from 0°/s² to a first maximum acceleration value of approximately 4000°/s² at time t1, whereafter the acceleration value drops as the head velocity drops, before finally coming to a rest at time t2.

Accordingly, when the processing device executes the fvHIT test protocol, the analysis device may continuously analyse the motion signals from the motion sensing device (which forms the basis for the curve) and determine the point in time t1 with a maximum acceleration value. Due to a time delay from determining the time t1 to actual presentation of an image by the display device, the analysis device may be configured to present a start signal in response to both the acceleration and velocity values exceeding respective thresholds. The start (initiation) signal may be presented at an earlier point in time than at the point in time with maximum acceleration value. In other words, a time delay may exist between said earlier point in time and said point in time with maximum acceleration value. The time delay may e.g., be at least 20 ms, such as 30 ms.

In response to determining the point in time t1, the display device may present an image, characterised by a property, for a pre-set time interval Δt starting from time t1 and until time t3. Afterwards, the image may disappear.

During the time of presentation of the image, the test subject has time to view the image and afterwards may provide a user input in form of an estimate of the property of said image.

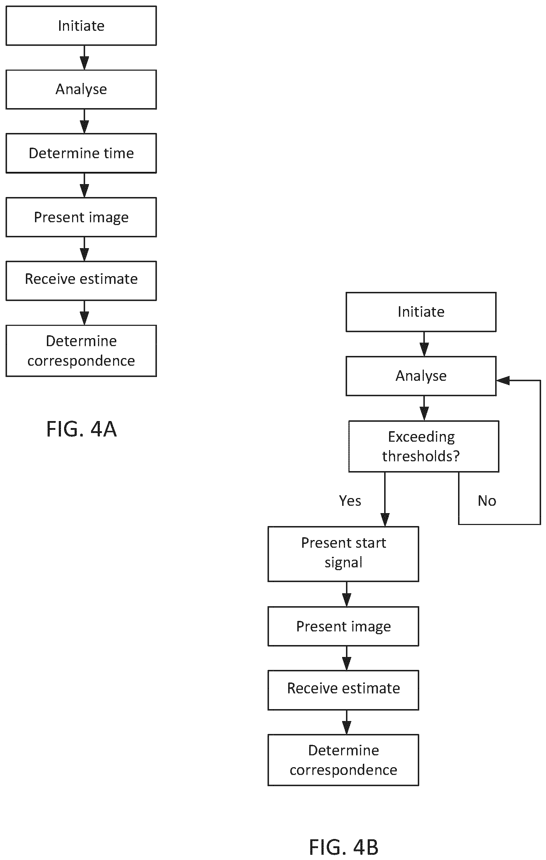

FIG. 4A shows an example of a flow diagram of a method of evaluating the function of the vestibular system of a test subject.

In particular, the method may relate to the above-mentioned fvHIT test protocol. After initiation ("Initiate") (e.g., by the user via the user input device), the test protocol may comprise a step of continuously analysing ("Analyse") the motion signals from the motion sensing device representing an acceleration of a rotation of the head of the test subject in at least one plane. The method may further comprise a step of determining ("Determine time") a point in time with the maximum acceleration value, at which point an image, characterised by a property, may be presented ("Present image") for a pre-set time interval by a display device starting from the determined point in time with maximum acceleration value. Afterwards, the method may comprise a step of receiving ("Receive estimate") a user input comprising an estimate of the property of said image via the user input device. Finally, the method may comprise a step of determining ("Determine correspondence") a correspondence between said property and said estimate of said property.

FIG. 4B shows an example of an additional flow diagram of a method of evaluating the function of the vestibular system of a test subject.

Compared to FIG. 4A, the flow diagram of FIG. 4B may additionally comprise the step of the analysis device presenting a start signal ("Present start signal") in response to both the acceleration and velocity values from the motion sensing devices being determined to exceed respective thresholds ("Exceeding thresholds?"). Otherwise, a start signal will not be presented, but instead the acceleration and velocity values of successive motion signals will be analysed.

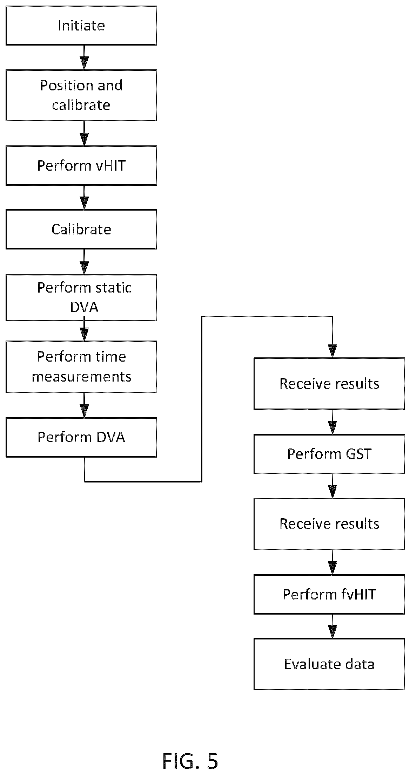

FIG. 5 shows an example of a flow diagram of a method of evaluating the function of the vestibular system of a test subject comprising a combination of test protocols.

One advantage of having a system configured to carry out a combination of test protocols is avoiding repetition. In other words, if you were to perform the DVA, GST, and fvHIT test protocols on the same day, you can copy the results of the static DVA test protocol (which evaluates the smallest optotype the test subject can see with head still) and of the VPT test protocol (which determines the fastest optotype the test subject can identify) to each of the DVA, GST, and fvHIT test protocols (e.g., at the same time).

Thereby, you don't have to perform the static DVA and VPT test protocols before each of the DVA, the GST, and the fvHIT test protocols.

Another advantage of having a system configured to carry out a combination of test protocols is that all the data measured and analysed may be compared in a single day, single report.

In FIG. 5, the combination of test protocols may comprise a vHIT test protocol, followed by a DVA test protocol and a GST test protocol, and finally by an fvHIT test protocol.

After initiation ("Initiate"), the method may comprise positioning the eye measurement device on the test subject and performing an eye calibration measurement ("Position and calibrate"). Then, the vHIT test protocol may be executed ("Perform vHIT") in the lateral plane, in the RALP plane, and in the LARP plane.

The motion sensing device may then be placed at the test subject's head (e.g., attached to the eye measurement device) and be calibrated ("Calibrate"). Thereby, the DVA test protocol may be executed, which involves the steps of performing static visual acuity measurements ("Perform static DVA"), i.e., determining the size of the image needed for the test subject to be able to clearly see the image at head still, followed by a step of performing visual processing time measurements (VPT test protocol) ("Perform time measurements"). Finally, the DVA test may be performed ("Perform DVA"), which may involve the user moving the test subject's head at 100°/s and inputting the test subject's estimate in relation to the image, until the smallest identifiable image threshold is reached for both movements in the lateral plane and in the vertical plane.

Following the DVA test protocol, the GST test protocol may be executed by the processing device initially receiving the results of the static DVA test and the visual processing time measurements ("Receive results"). Afterwards, the GST test may be performed ("Perform GST") by moving the test subject's head at increasing velocities and inputting the test subject's estimate in relation to the image, until measurements are carried out for velocity values above the threshold velocity value in the lateral plane and in the vertical plane.

Finally, the fvHIT test protocol may be executed by the processing device initially receiving the results of the static DVA test and the visual processing time measurements ("Receive results"). Afterwards, the fvHIT test may be performed ("Perform fvHIT") by moving the test subject's head at different accelerations and inputting the test subject's estimate in relation to the image (i.e., the property of the image), in both the lateral plane, the RALP plane, and the LARP plane.

After the combined test protocols have been executed, the user may evaluate the data ("Evaluate data") obtained from the vHIT, DVA, GST, and fvHIT tests (e.g., by use of the user interface) and produce a single report, which may be exported (e.g., to a patient data server) for later review.

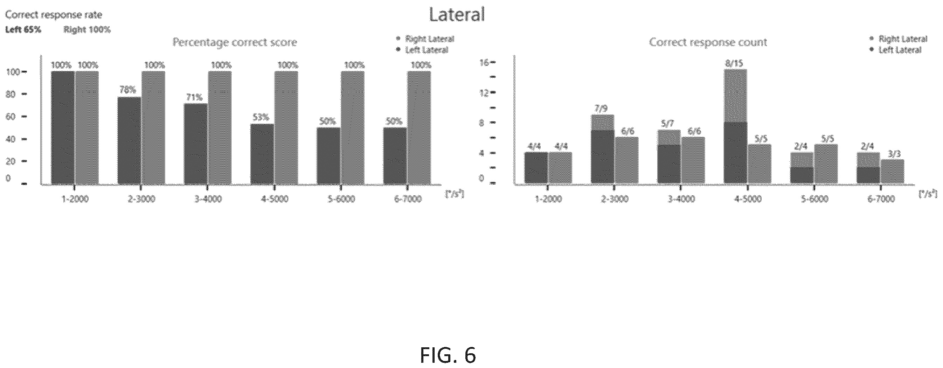

FIG. 6 shows an example of an average response rate calculation in the fvHIT test protocol for a lateral movement.

In FIG. 6, the left graph shows the percentage correct score (for the patient's estimate of the property of the image) as a function of head acceleration, °/s (divided into acceleration bins and right/left movements (i.e., for each acceleration bin, the left bar represents left movements, and the right bar represents right movements)).

The right graph shows the correct response count as a function of head acceleration, °/s (divided into acceleration bins and right/left movements (i.e., for each acceleration bin, the left bar represents left movements, and the right bar represents right movements)).

It is intended that the structural features of the system described above, either in the detailed description and/or in the claims, may be combined with steps of the method, when appropriately substituted by a corresponding process.

As used, the singular forms "a", "an", and "the" are intended to include the plural forms as well (i.e., to have the meaning "at least one"), unless expressly stated otherwise. It will be further understood that the terms "includes", "comprises", "including", and/or "comprising", when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

The claims are not intended to be limited to the aspects shown herein but are to be accorded the full scope consistent with the language of the claims, wherein reference to an element in the singular is not intended to mean "one and only one" unless specifically so stated, but rather "one or more". Unless specifically stated otherwise, the term "some" refers to one or more.

## Claims

1. System for evaluating the function of the vestibular system of a test subject, comprising
- an eye measurement device configured to record a pupil position, a velocity value, and/or an acceleration value of at least one of the eyes of the test subject,
- a motion sensing device configured to sense a movement and/or location of at least the head of the test subject and to provide at least one motion signal representing an acceleration value and/or a velocity value of a rotation of the head of the test subject in at least one plane,
- a display device configured to display at least one image,
- a user input device for receiving a user input,
- a processing device connected to the eye measurement device, the motion sensing device, and the display device,
- wherein the processing device is configured to execute at least one of a plurality of test protocols, where one test protocol comprises:
∘ continuously analysing the at least one motion signal from said motion sensing device and determining a point in time with a maximum acceleration value,
∘ presenting said image, **characterised by** a property, for a pre-set time interval by said display device starting from the determined point in time with maximum acceleration value,
∘ receiving a user input comprising an estimate of the property of said image via the user input device,
- wherein the processing device is configured to determine a correspondence between said property and said estimate of said property.

2. System according to claim 1, wherein the display device is configured to display said at least one image so that said image is visible to the test subject, and where said at least one image comprises an optotype.

3. System according to claim 1, wherein said property of said at least one image relates to an orientation, a size, a location, or a contrast level of said image.

4. System according to claim 1, wherein the step of continuously analysing the at least one motion signal from said motion sensing device and determining a point in time with the maximum acceleration value comprises the processing unit:
- comparing the acceleration value of each of the at least one motion signal with a pre-determined acceleration threshold,
- comparing the velocity value of each of the at least one motion signal with a pre-determined velocity threshold,
- presenting a start signal, in response to both the acceleration and velocity values exceed their respective thresholds.

5. System according to claim 4, wherein the step of presenting said image, **characterised by** a property, for a pre-set time interval by said display device starting from the determined point in time with maximum acceleration value comprises:
- presenting said image by the display device, in response to said start signal being presented, where the property of said image is determined by the executed test protocol.

6. System according to claim 1, wherein the step of receiving a user input comprising an estimate of the property of said image via the user input device comprises:
- receiving a response from the test subject or from the user of the system.

7. System according to claim 1, wherein the processing device is configured to score the percentage of correct estimates of said property at different accelerations of rotation of the head of the test subject in each respective plane.

8. System according to claim 1, wherein said processing device is configured to re-execute a specific test protocol in response to receiving an instruction in form of a user input via said user input device.

9. System according to claim 1, wherein the processing device is configured to execute one of the plurality of test protocols in response to receiving an instruction to execute a specific one of the plurality of test protocols via the user input device.

10. System according to claim 1, wherein said at least one plane comprises a lateral plane, a Right Anterior, Left Posterior (RALP) plane, and/or Left Anterior, Right Posterior (LARP) plane, where the lateral plane refers to a head rotation in a horizontal plane, the RALP plane refers to a head rotation in an orientation which best stimulates the Right Anterior, Left Posterior co-planar pair, and LARP refers to a head rotation in an orientation which best stimulates the Left Anterior, Right Posterior co-planar pair.

11. System according to claim 1, wherein the motion sensing device is configured to be attached to the eye measurement device.

12. System according to claim 1, wherein said display device comprises a refresh rate of at least 240 Hz.

13. System according to claim 1, wherein the user input device comprises a remote control, a keyboard, or a touch screen.

14. System according to claim 1, wherein said plurality of test protocols further comprises a Video Head Impulse Test (vHIT) protocol, a Dynamic Visual Acuity (DVA) test protocol, and/or a Gaze Stabilization (GST) test protocol.

15. System according to claim 1, wherein said processing device is configured to determine a visual focus of the test subject on said display device based on the recorded orientation of at least one of the eyes of the test subject.

16. System according to claim 1, wherein the eye measurement device comprises a camera for recording said at least one of the eyes of the test subject.

17. Method of evaluating the function of the vestibular system of a test subject, the method comprises
- executing at least one of a plurality of test protocols by a processing device, where one test protocol comprises:
∘ continuously analysing at least one motion signal from a motion sensing device representing an acceleration of a rotation of the head of the test subject in at least one plane,
∘ determining a point in time with a maximum acceleration value,
∘ presenting an image, **characterised by** a property, for a pre-set time interval by a display device starting from the determined point in time with maximum acceleration value, and
∘ receiving a user input comprising an estimate of the property of said image via a user input device,
- wherein the method further comprises determining a correspondence between said property and said estimate of said property.

18. A data processing system comprising a processing device and program code means for causing the processing device to perform at least some of the steps of the method of claim 17.

19. A computer program comprising instructions which, when the program is executed by a system according to any one of claims 1-16, cause the system to carry out the steps of the method according to claim 17.
